(19) Europäisches Patentamt
European Patent Office
Office européen des brevets

(11) EP 3 348 268 A1

(12) **EUROPEAN PATENT APPLICATION**
published in accordance with Art. 153(4) EPC

(43) Date of publication:
**18.07.2018 Bulletin 2018/29**

(21) Application number: **16844355.4**

(22) Date of filing: **06.09.2016**

(51) Int Cl.:
***A61K 31/519** (2006.01)* ***A61K 9/52** (2006.01)*
***A61P 25/18** (2006.01)* ***A61P 43/00** (2006.01)*

(86) International application number:
**PCT/JP2016/076198**

(87) International publication number:
**WO 2017/043494 (16.03.2017 Gazette 2017/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.09.2015 JP 2015175572**

(71) Applicant: **Nipro Corporation**
**Osaka-shi, Osaka 531-8510 (JP)**

(72) Inventors:
- **KOSAKA, Naoya**
**Osaka-shi**
**Osaka 531-8510 (JP)**
- **MIYANAGA, Naoyuki**
**Osaka-shi**
**Osaka 531-8510 (JP)**
- **HATSUSHIKA, Minoru**
**Osaka-shi**
**Osaka 531-8510 (JP)**

(74) Representative: **Vossius & Partner**
**Patentanwälte Rechtsanwälte mbB**
**Siebertstrasse 3**
**81675 München (DE)**

# (54) RISPERIDONE-CONTAINING MICROCAPSULE, METHOD FOR MANUFACTURING SAME AND RELEASE CONTROL METHOD

(57) The present invention provides a microcapsule containing a biodegradable polymer and risperidone and having a sustained release property as well as a method of producing the same. Provided are a microcapsule containing a biodegradable polymer and risperidone, in which the biodegradable polymer contained in the microcapsule has a molecular weight corresponding to 85% or more of an initial molecular weight (Mw) of the biodegradable polymer; a method of producing a microcapsule, comprising (A) a step of preparing a first phase by mixing a solution of a biodegradable polymer and a solution of risperidone which are prepared separately, (B) a step of, immediately after the preparation of the first phase, mixing the first phase with a second phase being an aqueous phase to prepare an emulsion, and (C) a step of subjecting the resulting emulsion to in-water drying; and a method of controlling release of risperidone from a microcapsule containing a biodegradable polymer and risperidone, wherein the biodegradable polymer contained in the microcapsule has an Mw corresponding to 85% or more of an initial molecular weight (Mw) of the biodegradable polymer.

FIG. 1

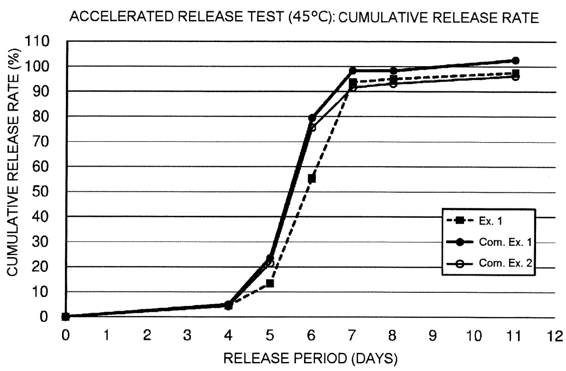


EP 3 348 268 A1

Printed by Jouve, 75001 PARIS (FR)

## Description

TECHNICAL FIELD

The present invention relates to a risperidone-containing microcapsule, a method for preparing the same and a method of controlling release of risperidone from the risperidone-containing microcapsule, and in particular relates to the microcapsule, the preparing method thereof and the release control method which are characterized in that a molecular weight of a biodegradable polymer in the microcapsule is within a specified range relative to an initial molecular weight thereof.

BACKGROUND ART

A variety of drug delivery systems, in which a drug useful for curing is encapsulated in a microcapsule of a biodegradable polymer or the like to prepare a sustained release preparation or a delayed release preparation, have been developed so far. For example, in a microcapsule using a biodegradable polymer such as a lactic acid-glycolic acid copolymer, a method of obtaining a microcapsule by dissolving, dispersing or emulsifying an encapsulating drug in a benzyl alcohol and ethyl acetate solution of a degradable polymer becoming a capsule shell and then washing and drying the solvent with an aqueous solution of ethyl acetate as a quenching liquid has been disclosed (Patent Document 1).

Meanwhile, risperidone which is a basic nucleophilic compound is an atypical antipsychotic agent called a serotonin-dopamine antagonist (SDA) used for curing of a schizophrenia and the like, and has an action of accelerating hydrolysis of a biodegradable polymer. Thus, for the purpose of controlling a molecular weight of a polymer for forming a shell of a microcapsule, a method of preparing an organic phase by mixing a solution of a biodegradable polymer dissolved in an ethyl acetate solution with a solution obtained by dissolving risperidone in benzyl alcohol, keeping the organic phase under a temperature of 25±5°C for a sufficient period of time for acceleration of a molecular weight loss corresponding to the starting material, and then performing an emulsification step, has been disclosed (Patent Document 2).

PRIOR ART DOCUMENT

Patent Documents

Patent Document 1: JP H09-505308 A

Patent Document 2: JP 2003-534268 A

SUMMARY OF THE INVENTION

PROBLEM TO BE SOLVED BY THE INVENTION

However, in the case of using risperidone as an active drug, in the method described in Patent Document 1, there is a problem that during a process of dissolving, dispersing or emulsifying risperidone in a benzyl alcohol and ethyl acetate solution of a biodegradable polymer, the biodegradable polymer such as a lactic acid-glycolic acid copolymer is decomposed, which makes it difficult to prepare a microcapsule comprising a polymer having a stable molecular weight. Further, it is described that from the viewpoint of preventing the active drug from being decomposed, a period of time for exposing the active drug to the polymer is as short as possible, that is, within 10 minutes, which, however, cannot be said to be satisfactory for the purpose of keeping a molecular weight of the biodegradable polymer during dissolution, dispersing and emulsification of the drug. Therefore, no consideration is taken with respect to the decomposition of the biodegradable polymer and an influence thereof on the release behavior. In a sustained release preparation containing risperidone, the molecular weight of the biodegradable polymer is one of factors for determining a drug release behavior, and therefore, it is strictly important to maintain a stable molecular weight in order to obtain a desired drug release behavior.

On the other hand, the method of Patent Document 2 is a complicated one, in which a necessary holding time should be calculated from a molecular weight of a biodegradable polymer as a starting material and be set appropriately in order to obtain a desired molecular weight by calculating. Further, in the case of obtaining a desired molecular weight by intentionally decomposing the polymer, there is a problem that it is difficult to control a decomposing process every time in the same manner, resulting in a large variation in a molecular weight between the preparation processes and

leading to a large variation in a release behavior of a preparation.

Thus, an object of the present invention is to provide a stable risperidone-containing microcapsule being capable of exhibiting a desired drug release behavior, a method of preparing the microcapsule and a method of controlling release of risperidone from the microcapsule.

MEANS TO SOLVE THE PROBLEM

The inventors of the present invention have found that in a microcapsule of a biodegradable polymer containing risperidone, a desired risperidone release behavior can be achieved by allowing the biodegradable polymer to have a molecular weight corresponding to 85% or more of an initial molecular weight of the biodegradable polymer, and have completed the present invention. The inventors of the present invention have found that by adjusting a decreasing rate of a molecular weight of the biodegradable polymer to be within a predetermined range, a desired release behavior of risperidone can be obtained without being restricted by a solvent for dissolving the biodegradable polymer.

Namely, the present invention relates to:

[1] a microcapsule comprising a biodegradable polymer and risperidone, wherein the biodegradable polymer in the microcapsule has a molecular weight (Mw) corresponding to 85% or more, preferably more than 90% of an initial molecular weight (Mw) of the biodegradable polymer,

[2] the microcapsule of the above [1], wherein the initial molecular weight (Mw) of the biodegradable polymer is not less than 50,000 and less than 100,000, preferably not less than 60,000 and less than 100,000, more preferably not less than 70,000 and less than 100,000,

[3] a method for preparing a microcapsule comprising a biodegradable polymer and risperidone, the method comprising:

(A) a step of preparing a first phase by mixing a solution of the biodegradable polymer and a solution of the risperidone which have been prepared separately,

(B) a step of, immediately after the preparation of the first phase, mixing the first phase with a second phase being an aqueous phase to prepare an emulsion, and

(C) a step of subjecting the resulting emulsion to in-water drying,

[4] the method of the above [3], wherein a molecular weight (Mw) of the biodegradable polymer in the microcapsule is a molecular weight (Mw) corresponding to 85% or more, preferably more than 90% of an initial molecular weight (Mw) of the biodegradable polymer,

[5] the method of the above [3] or [4], wherein the initial molecular weight (Mw) of the biodegradable polymer is not less than 50,000 and less than 100,000, preferably not less than 60,000 and less than 100,000, more preferably not less than 70,000 and less than 100,000,

[6] the method of any of the above [3] to [5], wherein the mixing in the step (A) is performed using a Static Mixer.

Further the present invention is directed to a method of controlling a release behavior of a microcapsule comprising a biodegradable polymer and risperidone.

Namely the present invention also relates to:

[7] a method of controlling release of risperidone from a microcapsule comprising a biodegradable polymer and risperidone, wherein a molecular weight (Mw) of the biodegradable polymer in the microcapsule is allowed to be a molecular weight (Mw) corresponding to 85% or more of an initial molecular weight (Mw) of the biodegradable polymer, and

[8] a method of controlling release of risperidone from a microcapsule comprising a biodegradable polymer and risperidone, the method comprising:

(A) a step of preparing a first phase by mixing a solution of the biodegradable polymer and a solution of the risperidone which have been prepared separately,

(B) a step of, immediately after the preparation of the first phase, mixing the first phase with a second phase being an aqueous phase to prepare an emulsion, and

(C) a step of subjecting the resulting emulsion to in-water drying.

EFFECTS OF THE INVENTION

According to the present invention, a desired release behavior of risperidone can be obtained by adjusting a

decreasing rate of a molecular weight of the biodegradable polymer to be within a predetermined range. Further, according to the present invention, by preparing a first phase by mixing a solution of a biodegradable polymer and a solution of risperidone which have been prepared separately and immediately thereafter, emulsifying the mixture, a stable microcapsule containing risperidone can be prepared easily with a molecular weight of the biodegradable polymer being maintained to be as stable as possible.

BRIEF DESCRIPTION OF THE DRAWING

FIG. 1 is a graph showing a cumulative release rate (%) of risperidone.

EMBODIMENT FOR CARRYING OUT THE INVENTION

In the present invention, the microcapsule comprising a biodegradable polymer and risperidone is characterized in that the biodegradable polymer in the microcapsule has a molecular weight (Mw) corresponding to 85% or more of an initial molecular weight (Mw) of the biodegradable polymer.

Herein, the "microcapsule" means a particle containing a polymer acting as a matrix or a binder of the particle. The microcapsule may comprise an active agent or other substance dispersed or dissolved in a microcapsule polymer matrix. The polymer is preferably biodegradable and biocompatible. The "biodegradable" means a property of being easily decomposed and metabolized in a body, and the "biocompatible" means a property of being not poisonous and being acceptable pharmaceutically.

Risperidone is a benzisoxazole derivative of a chemical name: 3-{2-[4-(6-fluoro-1,2-benzisoxazole-3-yl)piperidino]ethyl}-6,7,8,9-tetrahydro-2-methyl-4H-pyrido[1,2-a]pyrimidine-4-on represented by the formula:

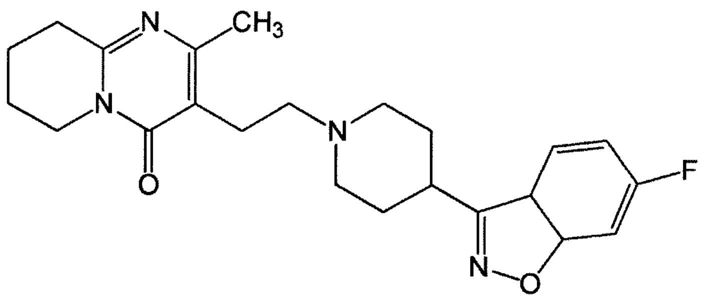

.

Risperidone is an atypical antipsychotic agent called a serotonin-dopamine antagonist (SDA), and exhibits an antagonism against both of a dopamine D2 receptor and a serotonin 5-HT2A receptor. The both antagonisms show an action of improving both of positive symptom and negative symptom of schizophrenia.

Examples of the biodegradable polymer include polyglycolic acid, poly-D, L-lactic acid, poly-L-lactic acid and copolymers thereof and salts thereof, and a lactic acid-glycolic acid copolymer (poly(DL-lactic-co-glicolide)) is preferable. These can be prepared by a well-known method, and various commercially available ones can also be used.

Examples of a salt of a lactic acid-glycolic acid copolymer include salts and complex salts thereof with inorganic bases including alkaline metals such as sodium and potassium and alkali earth metals such as calcium and magnesium, organic bases such as organic amines including triethylamine and basic amines including arginine, or transition metals such as zinc, iron and copper.

The initial molecular weight of the biodegradable polymer is usually preferably 50,000 or more, more preferably 60,000 or more, further preferably 70,000 or more in a weight-average molecular weight Mw. When the initial molecular weight (Mw) of the biodegradable polymer is 50,000 or more, a desired drug release property tends to be obtained easily. The initial molecular weight (Mw) of the biodegradable polymer is preferably less than 100,000. If the initial molecular weight (Mw) of the biodegradable polymer exceeds 100,000, there is a tendency that it takes a time to synthesize the biodegradable polymer itself and solubility thereof in a solvent is deteriorated.

The molecular weight (Mw) of the biodegradable polymer in the microcapsule is 85% or more, more preferably more than 90% of the above-mentioned initial molecular weight (Mw). When a difference between the molecular weight of the biodegradable polymer in the microcapsule and the initial molecular weight is made small, a variation of a molecular weight in the production process can be inhibited and a variation of a release behavior of a preparation can also be reduced.

A content of risperidone in the microcapsule is preferably 1 to 60% by mass, more preferably 35 to 45% by mass. When the risperidone content is less than 1% by mass based on the weight of the microcapsule, it is necessary to administrate a large amount of microcapsule preparation in order to satisfy a clinical dose. When the risperidone

content exceeds 60% by mass based on the weight of the microcapsule, there is a problem that it takes a time to dissolve risperidone, it is necessary to increase an amount of a solvent not to leave risperidone undissolved, and emulsification needs a long period of time.

In the present invention, the method of producing a microcapsule comprising a biodegradable polymer and risperidone comprises (A) a step of preparing a first phase by mixing a solution of a biodegradable polymer and a solution of risperidone which have been prepared separately, (B) a step of, immediately after the preparation of the first phase, mixing the first phase with a second phase being an aqueous phase to prepare an emulsion, and (C) a step of subjecting the resulting emulsion to in-water drying.

Examples of a solvent to be used for preparing the solution of a biodegradable polymer include ethyl acetate, dichloromethane, cyclohexane, chloroform, tetrahydrofuran and the like, and ethyl acetate is preferable from the viewpoint that its level in the guide line on a residual solvent of pharmaceuticals is as low as class 3. Examples of a solvent to be used for preparing the solution of risperidone include benzyl alcohol, methanol, ethanol and the like, and benzyl alcohol is preferable from the viewpoint of solubility.

The step (A) is not limited particularly. Meanwhile, it is preferable to prepare an ethyl acetate solution of a biodegradable polymer and a benzyl alcohol solution of risperidone separately and immediately before the step (B), mix the both solutions to prepare the first phase. Thus, an interaction between the biodegradable polymer and the risperidone can be inhibited to the utmost.

It is preferable to use the first phase obtained by mixing the solution of a biodegradable polymer and the solution of risperidone immediately after the preparation thereof. Therefore, it is preferable to prepare the second phase being an aqueous phase before the preparation of the first phase and prepare the first phase just before the emulsification of the step (B). Thus, an interaction between the biodegradable polymer and the risperidone can be inhibited to the utmost, and decomposition of the biodegradable polymer can be inhibited.

Herein, to use "immediately after the preparation" means that a time of exposure of risperidone to the biodegradable polymer is as short as possible in order to inhibit an interaction between the biodegradable polymer and the risperidone to the utmost. This means that the step (B) follows, for example, within at least 1.5 minutes, preferably within one minute, or more preferably the step from the preparation of the first phase up to the step (B) is performed continuously.

In the step (A), for the mixing of the solution of a biodegradable polymer and the solution of risperidone, a usual mixing means can be used, and use of a Static Mixer is preferable since the step after the mixing up to the step (B) can be performed continuously. In this case, cooling of the first phase may be performed during the mixing of the two solutions with the Static Mixer by providing a cooling means on the Static Mixer (for example, providing, outside the Static Mixer, a tank in which cooling water is flowed).

The second phase in the step (B) is an aqueous phase, and it is preferable to add a hydrophilic colloid or a surfactant to stabilize the emulsion and adjust a size of the microcapsule in the emulsion. A usable compound as a hydrophilic colloid or a surfactant is not limited particularly, and examples thereof include polyvinyl alcohol, carboxymethyl cellulose, gelatin, polyvinyl pyrrolidone, Tween80, Tween20 and the like.

A concentration of the hydrophilic colloid or the surfactant in the second phase should be enough for stabilization of the emulsion, and can affect a final size of the microcapsule. The concentration of the hydrophilic colloid or the surfactant in the second phase varies with kind of the hydrophilic colloid or the surfactant, and in the case of polyvinyl alcohol, is preferably not less than 0.01% by mass, more preferably not less than 0.1% by mass and preferably not more than 10% by mass, more preferably not more than 5% by mass. When the concentration of polyvinyl alcohol is less than 0.01% by mass, there is a tendency that particles of the microcapsule agglomerate with each other and become a mass. When the concentration exceeds 10% by mass, bubbles generated during the emulsification cover a liquid surface at the time of in-water drying and prevent evaporation of an organic solvent, which may increase an amount of a remaining solvent in the microcapsule.

Further in one embodiment of the present invention, it is preferable to cool at least either one of the first phase and the second phase. Each component and a solution before preparing the first phase may be cooled, or cooling may be conducted during the preparation process of the first phase or the second phase may be cooled after the preparation thereof. By cooling of either of these phases, there is a tendency that the likelihood of decrease in the molecular weight of the biodegradable polymer after the mixing in the step (B) can be prevented. Herein, "cooling" means temperature decrease to lower than 15°C, preferably 15°C or lower, more preferably 5°C or lower.

Further in one embodiment of the present invention, it is preferable that the mixing in the step (B) is performed continuously after the above-mentioned step (A) by means of an emulsifier, for example, a Static Mixer, a homo-mixer or a high pressure homogenizer. By conducting the step (B) continuously, decomposition of the biodegradable polymer can be inhibited more effectively.

Examples of the step of drying the emulsion include methods of evaporating an organic solvent such as in-water drying, freeze-drying and drying under reduced pressure. More preferably, the step of drying the emulsion is in-water drying.

In one embodiment of the present invention, it is preferable to perform all the steps, particularly the step (A) of

preparing the first phase and the step (B) of mixing the second phase 2 and the first phase containing risperidone preferably under cooling, further preferably at 15°C or lower, more preferably 5°C or lower. In the case of performing each step at a temperature higher than 15°C, there may be a case where an effect of maintaining the molecular weight of the biodegradable polymer of the present invention cannot be exhibited enough. Further, in the case of in-water drying in the step (C) of drying the emulsion, it is preferable to perform the drying at 15°C or lower.

Herein, "desired release behavior" means that in the delayed release preparation containing the microcapsule of the present invention, starting of release after administration is slow. Namely, in the case of using the microcapsule containing the biodegradable polymer and risperidone as an injection for curing of schizophrenia, it is desired to decrease the number of administrations and improve a sustained release property. When starting of release is slow, sudden elevation of a concentration in blood can be inhibited and a risk of generating a side effect can be reduced.

The microcapsule of the present invention can be formed into a preparation usable for medical application to an injector by conducting filtration using a proper sieve before and after the drying or at least after the drying.

In the case of using the microcapsule of the present invention as an injection, it can be prepared being suspended in an appropriate pharmaceutically acceptable suspension which has been known in a related technical field.

## EXAMPLE

The present invention is then explained below in detail by means of Examples and Comparative Examples, but is not limited to these Examples.

### Example 1

A solution (a) of 3.0 g of risperidone in 9.51 g of benzyl alcohol and a solution (b) of 4.87 g of a lactic acid-glycolic acid copolymer (lactic acid : glycolic acid = 3:1, weight-average molecular weight: 74,000/PLGA) in 22.5 g of ethyl acetate were prepared. A second phase being a solution obtained by adding ethyl acetate to an aqueous solution of 1% by mass of polyvinyl alcohol (PVA) to become 6.5% by mass was prepared previously. The solution (a) and the solution (b) were mixed to obtain a first phase, and immediately after the mixing (within 1.5 minutes), the first phase was fed at a rate of 10 mL/min and the second phase was fed at a rate of 50 mL/min to a PIPELINE-HOMO MIXER (manufactured by PRIMIX CORPORATION), followed by emulsification to obtain an O/W emulsion (emulsification time: 4 minutes).

Next, 2.5 L of 2.5% aqueous solution of ethyl acetate was added to the resulting emulsion, followed by 3-hour stirring at 10°C with a propeller stirrer and in-water drying for evaporation of the solvent.

After the evaporation of the solvent, the mixture was sieved with a 150 μm sieve to collect a sieved solution which was then sieved with a 25 μm sieve. The obtained residual on the sieve was collected and washed with 0.5 L of water, followed by freeze-drying and further sieving with a 425 μm sieve to obtain 3.8 g of a microcapsule.

### Example 2

A solution (a) of 3.0 g of risperidone in 9.51 g of benzyl alcohol and a solution (b) of 4.87 g of a lactic acid-glycolic acid copolymer (lactic acid : glycolic acid = 3:1, weight-average molecular weight: 74,000/PLGA) in 22.5 g of ethyl acetate were prepared. A second phase being a solution obtained by adding ethyl acetate to an aqueous solution of 1% by mass of polyvinyl alcohol (PVA) to become 6.5% by mass was prepared previously. The solution (a) was fed at a rate of 2.7 mL/min and the solution (b) was fed at a rate of 7.3 mL/min to a Static Mixer (manufactured by NORITAKE CO., LIMITED) connected to a PIPELINE-HOMO MIXER, and while mixing the solutions, a first phase being the mixture was fed to the PIPELINE-HOMO MIXER (manufactured by PRIMIX CORPORATION), followed continuously by emulsification with the second phase to obtain an O/W emulsion (emulsification time: 4 minutes). The feeding speeds of the first phase and the second phase were 10 mL/min and 50 mL/min, respectively.

Next, 2.5 L of 2.5% aqueous solution of ethyl acetate was added to the resulting emulsion, followed by 3-hour stirring at 10°C with a propeller stirrer and in-water drying for evaporation of the solvent.

After the evaporation of the solvent, the mixture was sieved with a 150 μm sieve to collect a sieved solution which was then sieved with a 25 μm sieve. The obtained remaining substance on the sieve was collected and washed with 0.5 L of water, followed by freeze-drying and further sieving with a 425 μm sieve to obtain 3.0 g of a microcapsule.

### Comparative Example 1

A second phase which was a solution obtained by adding ethyl acetate to an aqueous solution of 1% by mass of polyvinyl alcohol (PVA) to become 6.5% by mass was prepared. A first phase which was a solution mixture comprising 9.51 g of benzyl alcohol and 22.5 g of ethyl acetate containing 3.0 g of risperidone and 4.87 g of a lactic acid-glycolic

acid copolymer (lactic acid : glycolic acid = 3:1, weight-average molecular weight: 93,000/PLGA) was prepared. After having been allowed to stand at room temperature for three hours, the first phase was emulsified with the second phase with a PIPELINE-HOMO MIXER (manufactured by PRIMIX CORPORATION) to obtain an O/W emulsion (emulsification time: 4 minutes). The feeding speeds of the first phase and the second phase were 10 mL/min and 50 mL/min, respectively.

Next, 2.5 L of 2.5% aqueous solution of ethyl acetate was added to the resulting emulsion, followed by 3-hour stirring at 10°C with a propeller stirrer and in-water drying for evaporation of the solvent.

After the evaporation of the solvent, the mixture was sieved with a 150 μm sieve to collect a sieved solution which was then sieved with a 25 μm sieve. The obtained remaining substance on the sieve was collected and washed with 0.5 L of water, followed by freeze-drying and further sieving with a 425 μm sieve to obtain 3.0 g of a microcapsule.

Comparative Example 2

A second phase which was a solution obtained by adding ethyl acetate to an aqueous solution of 1% by mass of polyvinyl alcohol (PVA) to become 6.5% by mass was prepared. A first phase which was a solution mixture comprising 9.51 g of benzyl alcohol and 22.5 g of ethyl acetate containing 3.0 g of risperidone and 4.87 g of a lactic acid-glycolic acid copolymer (lactic acid : glycolic acid = 3:1, weight-average molecular weight: 86,000/PLGA) was prepared. After having been allowed to stand at room temperature for three hours, the first phase was emulsified with the second phase with a PIPELINE-HOMO MIXER (manufactured by PRIMIX CORPORATION) to obtain an O/W emulsion (emulsification time: 4 minutes). The feeding speeds of the first phase and the second phase were 10 mL/min and 50 mL/min, respectively.

Next, 2.5 L of 2.5% aqueous solution of ethyl acetate to the resulting emulsion, followed by 3-hour stirring at 10°C with a propeller stirrer and in-water drying for evaporation of the solvent.

After the evaporation of the solvent, the mixture was sieved with a 150 μm sieve to collect a sieved solution which was then sieved with a 25 μm sieve. The obtained remaining substance on the sieve was collected, followed by freeze-drying and further sieving with a 425 μm sieve to obtain 3.6 g of a microcapsule.

Test Example 1

Weight-average molecular weights of the lactic acid-glycolic acid copolymers in the microcapsules obtained in Examples 1 and 2 and Comparative Examples 1 and 2 were measured by the following method. About 40 mg of microcapsule was dissolved in 5 mL of tetrahydrofuran to obtain a sample solution. Subsequently, 10 μL of the sample solution was put in a chromatography equipped with molecular sieving columns (manufactured by TOSO CORPORATION: TSK-GEL G6000$H_{XL}$, G5000$H_{XL}$, G4000$H_{XL}$, G2500$H_{XL}$, G1000$H_{XL}$, column temperature: 40°C), followed by development at a flow rate of 0.8 mL/min in a tetrahydrofuran mobile phase to measure a weight-average molecular weight. The decreasing rates of the respective molecular weights are shown in Table 1. The decreasing rate of the molecular weight was calculated by the following formula.

Decreasing rate of molecular weight (%) = (Weight-average molecular weight of PLGA as a starting material − Weight-average molecular weight of PLGA in microcapsule) / Weight-average molecular weight of PLGA as a starting material × 100

Table 1

| | Example | | Com. Ex. | |
|---|---|---|---|---|
| | 1 | 2 | 1 | 2 |
| Decreasing rate of molecular weight of PLGA (%) | 13.7 | 9.9 | 34.5 | 29.4 |

Test Example 2

With respect to the microcapsules obtained in Example 1 and Comparative Examples 1 and 2, risperidone release characteristic was evaluated by an accelerated release test at 45°C. The microcapsule was put in a test solution (pH: 7.4), followed by shaking in a thermostatic bath of 45°C. Four days, five days, six days, seven days, eight days and

eleven days after starting of the shaking, a part of the test solution was collected, and a content of risperidone in the test solution was measured with an ultraviolet absorption spectrophotometer. The results are shown in Table 2 and FIG. 1.

Table 2

| Release period (Days) | Cumulative Release Rate (%) | | |
|---|---|---|---|
| | Ex. 1 | Com. Ex. 1 | Com. Ex. 2 |
| 0 | 0 | 0 | 0 |
| 4 | 4.45 | 5.00 | 4.39 |
| 5 | 13.39 | 23.52 | 21.40 |
| 6 | 55.23 | 79.46 | 75.58 |
| 7 | 93.77 | 98.36 | 91.63 |
| 8 | 95.07 | 98.31 | 93.20 |
| 11 | 97.56 | 102.60 | 96.25 |

In Example 1, starting-up of a release behavior is slow, which is considered to be an ideal behavior. On the other hand, release behaviors of Comparative Examples 1 and 2 show sudden starting-up and there is a concern about a side effect resulting from a sudden elevation of a concentration in blood. Further in the case where PLGA is greatly degraded, a release behavior shifts toward a earlier release period as compared with a case of less degradation. Therefore, management of an elapsed time after the preparation of the first phase is important. By conducting the emulsification immediately after the preparation of the first phase, PLGA is less degraded and there is less variation in release behavior between the preparations.

## Claims

1. A microcapsule comprising a biodegradable polymer and risperidone, wherein
   the biodegradable polymer in the microcapsule has a molecular weight (Mw) corresponding to 85% or more of an initial molecular weight (Mw) of the biodegradable polymer.

2. The microcapsule of claim 1, wherein the initial molecular weight (Mw) of the biodegradable polymer is not less than 50,000 and less than 100,000.

3. A method for preparing a microcapsule comprising a biodegradable polymer and risperidone, the method comprising:

   (A) a step of preparing a first phase by mixing a solution of the biodegradable polymer and a solution of the risperidone which have been prepared separately,
   (B) a step of, immediately after the preparation of the first phase, mixing the first phase with a second phase being an aqueous phase to prepare an emulsion, and
   (C) a step of subjecting the resulting emulsion to in-water drying.

4. The method of claim 3, wherein a molecular weight (Mw) of the biodegradable polymer in the microcapsule is a molecular weight (Mw) corresponding to 85% or more of an initial molecular weight (Mw) of the biodegradable polymer.

5. The method of claim 3 or 4, wherein the initial molecular weight (Mw) of the biodegradable polymer is not less than 50,000 and less than 100,000.

6. The method of any one of claims 3 to 5, wherein the mixing in the step (A) is performed using a Static Mixer.

7. A method of controlling release of risperidone from a microcapsule comprising a biodegradable polymer and risperidone, wherein a molecular weight (Mw) of the biodegradable polymer in the microcapsule is allowed to be a molecular weight (Mw) corresponding to 85% or more of an initial molecular weight (Mw) of the biodegradable polymer.

8. A method of controlling release of risperidone from a microcapsule comprising a biodegradable polymer and risperidone, the method comprising:

(A) a step of preparing a first phase by mixing a solution of the biodegradable polymer and a solution of the risperidone which have been prepared separately,
(B) a step of, immediately after the preparation of the first phase, mixing the first phase with a second phase of an aqueous phase to prepare an emulsion, and
(C) a step of subjecting the resulting emulsion to in-water drying.

**9.** The method of claim 8, wherein the mixing in the step (A) is performed using a Static Mixer.

FIG. 1

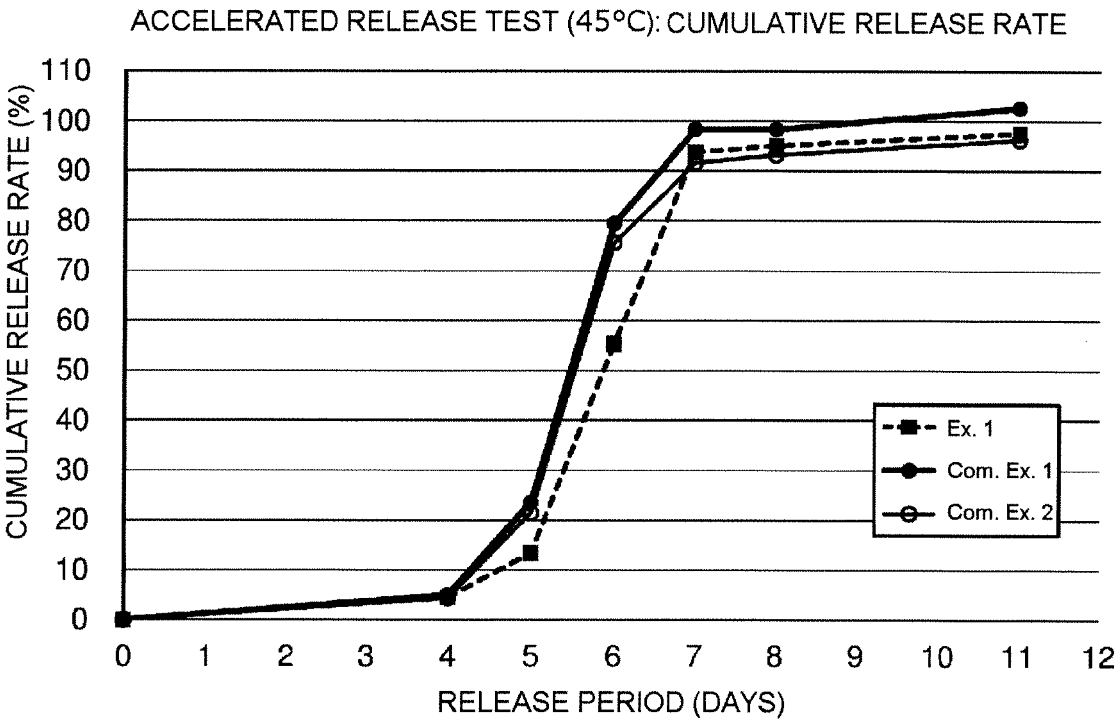
ACCELERATED RELEASE TEST (45°C): CUMULATIVE RELEASE RATE
CUMULATIVE RELEASE RATE (%)
110
100
90
80
70
60
50
40
30
20
10
0
0
1
2
3
4
5
6
7
8
9
10
11
12
RELEASE PERIOD (DAYS)
Ex. 1
Com. Ex. 1
Com. Ex. 2

## INTERNATIONAL SEARCH REPORT

International application No.
PCT/JP2016/076198

A. CLASSIFICATION OF SUBJECT MATTER
*A61K31/519*(2006.01)i, *A61K9/52*(2006.01)i, *A61P25/18*(2006.01)i, *A61P43/00* (2006.01)i

According to International Patent Classification (IPC) or to both national classification and IPC

B. FIELDS SEARCHED

Minimum documentation searched (classification system followed by classification symbols)
A61K31/519, A61K9/52, A61P25/18, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/WPIDS/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 2003-534268 A (Alkermes Controlled Therapeutics Inc. II), 18 November 2003 (18.11.2003), claims; paragraphs [0016], [0022] to [0031], [0046] to [0049], [0053] to [0054]; fig. 1 to 3 & US 6264987 B1 claims; column 3, lines 33 to 37; examples 1, 4, 6; fig. 1 to 3 & WO 2001/089482 A2 & EP 1282404 A2 & DE 60134640 D & AU 5546501 A & CA 2405787 A & AT 399535 T & DK 1282404 T & ES 2305071 T & PT 1282404 E & CY 1110403 T | 1-9 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>27 October 2016 (27.10.16) | Date of mailing of the international search report<br>08 November 2016 (08.11.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.
PCT/JP2016/076198

C (Continuation). DOCUMENTS CONSIDERED TO BE RELEVANT

| Category* | Citation of document, with indication, where appropriate, of the relevant passages | Relevant to claim No. |
|---|---|---|
| X<br>A | SU, Zhengxing et al., Effects of Formulation Parameters on Encapsulation Efficiency and Release Behavior of Risperidone Poly(D,L-lactide-co-glycolide) Microsphere, Chem. Pharm. Bull., 2009, Vol. 57, No. 11, pp. 1251-1256, Abstract, Fig. 2 | 1-2<br>3-9 |
| P,X | JP 2016-102093 A (Nipro Corp.), 02 June 2016 (02.06.2016), paragraphs [0036] to [0039] (Family: none) | 1-9 |
| A | JP 2014-512396 A (Shandong Luye Pharmaceutical Co., Ltd.), 22 May 2014 (22.05.2014), & US 2014/0050799 A1 & WO 2012/146052 A1 & EP 2701687 A1 & CN 103338752 A & AU 2012248038 A & CA 2832663 A & KR 10-2014-0026481 A & RU 2013147949 A & HK 1185560 A | 1-9 |
| A | JP 9-505308 A (Alkermes Controlled Therapeutics Inc. II), 27 May 1997 (27.05.1997), & US 5650173 A & WO 1995/013799 A1 & EP 729353 A1 & DE 69429820 D & AU 1101095 A & CA 2176716 A & AT 212830 T & DK 729353 T & ES 2172574 T & PT 729353 E | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H09505308 A
- JP 2003534268 A